# EUROPEAN PATENT APPLICATION

(11) **EP 2 832 741 A1**
(43) Date of publication of application: **04.02.2015**
(21) Application number: 13306099.6
(22) Date of filing: 30.07.2013
(51) Int. Cl.: C07K 1/30

(54) **Method of crystallization of membrane proteins and to compositions, devices and kits to conduct such method**

(71) Applicant: COMMISSARIAT A L'ENERGIE ATOMIQUE ET AUX ENERGIES ALTERNATIVES, 75015 Paris (FR)
(72) Inventor: Gordeliy, Valentin, 38220 Laffrey (FR); Round, Ekaterina, 38134 Saint Joseph-de-Rivière (FR); Borshchevskiy, Valentin, 52428 Jülich (DE); Gushchin, Ivan, 52428 Jülich (DE); Polovinkin, Vitaly, 38610 Gières (FR)
(74) Representative: Bernstein, Claire Jacqueline

(57) **Abstract**

The present invention relates to a new method of crystallization of membrane proteins and to compositions, devices and kits to conduct such method.

## Description

The present invention relates to a new method of crystallization of membrane proteins and to compositions and kits to conduct such method.

Membrane proteins (MP or MPs) perform wide variety of functions including transport of different molecules and ions inside the cell and back to the extracellular space, enzymatic action, participation in signal transmission through the membrane, action as receptors for hormones and neurotransmitters, etc. This broad range of functions makes MPs very important for operation of the whole organism. Any changes in physicochemical properties of MP can lead to the variety of pathological processes in the cell. MPs account for ∼ 60-70 % of known targets of the drugs presented at the pharmaceutical market [Rivna, A.W. et al (2009) Top Med Chem 4, 15-51]. Development of new effective drugs needs the fundamental understanding of molecular mechanism of MPs action, which can be clarified by knowledge of the structures of MPs at atomic resolution. Currently the most detailed information about the structure of macromolecules can be obtained using X-ray analysis of their three-dimensional (3D) crystals. However, the growing of 3D crystals of MPs is a big challenge due to the difficulties with their overexpression and purification, instability outside the membrane environment and amphiphilic nature, which complicates significantly their crystallization. In addition, crystallization process itself is a great (if not the greatest) challenge. As a result, after 27 years of investigations of 3D structures of MPs (since the first published structure of photosynthetic reaction center (PSRC) from *Rhodopseudomonas viridis* in 1985 [Deisenhofer,J. et al. (1985) Nature 318, 618-624]), only 343 of more than 82000 registered high-resolution protein structures represent the unique MPs [Database "Membrane Proteins of Known Structure" http://blanco.biomol.uci.edu/mpstruc/listAll/list.; RSCB Protein Data Bank http://www.pdb.org/pdb/home].

Classical approach to crystallization of membrane proteins: MPs are amphiphilic molecules and cannot be dissolved either in water or in organic solvents without protein unfolding. The hydrophobic part of MP interacts with hydrocarbon chains of lipids and contains mainly non-polar amino acids while the hydrophilic one interacts with water molecules on the both sides of the membrane and with the polar heads of the lipids. Hydrophobic parts of MPs need to be surrounded by special amphiphilic molecules to be transferred into dissolved state in aqueous environment. This process is called solubilization of MPs and is conducted with detergents which are amphiphilic molecules that form a belt around the hydrophobic part of MP [Garavito, R.M. et al. (2001) J Bio Chem 276, 32403-32406].

In such solubilized state, some MPs can then be crystallized using standard methods of crystallization for water-soluble proteins. The general principle of these methods is to obtain a supersaturated solution of the protein by slow variation of the parameters of the crystallization system. This solution promotes nucleation of the protein crystals and their further growing [Reiss-Husson, F. (1999) Crystallization of membrane proteins. In: Crystallization of nucleic acids and proteins. A practical approach (Ducruix, A., Giege, R., eds.). Oxford University Press*].* Different techniques for this standard crystallization approach are known. The search for crystallization conditions, which lead to protein crystals formation, is usually very difficult and labor-extensive and very far from being always successful.

One of the major problems in crystallization of MPs by standard approach is the selection of an appropriate detergent. The choice of detergent should be implemented in according with several strict requirements. The detergent concerned should stabilize the native conformation of the MP in monodisperse form, i.e. simulate the natural environment for the protein. Usually the detergents with long hydrocarbon chains are good to mimic this lipid environment since their form and organization are close to ones of natural lipids [Iwata, S. (2003) Methods and Results in Crystallization of Membrane Proteins. International Unversity Line.*;* Prive, G.G. (2007) Methods 41, 388-397]. From the other hand, the detergent has to enable protein-protein contacts in the packed crystal. Therefore the detergent molecule should not be very large to avoid the blocking of polar regions interactions of two neighboring MPs by large detergent belt [Nollert, P. (2005) Prog Biophys Mol Biol 88, 339-357]. Thereby it was noticed that the detergents, which seem to be suitable for crystallization of MPs, usually are less suitable for their stability in the solution [Michel, H. (1991) General and practical aspects in membrane proteins crystallization. In: Crystallization of Membrane Proteins (Michel, H., ed.). CRC, Boca Raton, FL, 73-88]. In addition, the crystallization conditions chosen should prevent detrimental phase separations during crystal growth, which also imposes constraints on the choice of the detergent [Michel, H. (1991) General and practical aspects in membrane proteins crystallization. In: Crystallization of Membrane Proteins (Michel, H., ed.). CRC, Boca Raton, FL, 73-882]. Taking into account these requirements, the large variety of detergents of different concentrations should be tested for each protein concerned to make the optimal choice of crystallization conditions.

Taking into account the importance of lipids for stabilization and crystallization of MPs, Landau and Rosenbusch proposed in 1996 an alternative approach to crystallization of MPs called crystallization in bicontinuous lipidic cubic phase (LCP) [Landau, E.M., Rosenbusch, J.P. (1996) Proc. Natl. Acad. Sci. USA 93, 14532-14535]: in this approach, the detergent solution of MP is introduced in a lipid bilayer which is in a liquid crystalline phase (mesophase) and is used like a matrix for crystallization of MPs. One advantage of the crystallization of MPs in lipidic mesophases lays in the fact that mesophase provides a more native-like membrane environment for proteins as opposed to a inappropriate environment associated with detergent micelles.

Nevertheless, despite some very important breakthroughs (bacteriorhodopsin, GPCRs [Pebay-Peyroula et al. (1997) Science 277, 1676-1681; Cherezov et al. (2007) Science 318, 1258]), this approach has not led to a significant progress in structural biology of membrane proteins - the number of new structures of MPs is still growing quite slowly. A drawback of this approach is that it does not solve the problem of solubilization of MPs with the detergents. This first step involves the selection of detergents to have more room for further crystallization; this makes the first step even more laborious. It is not an exceptional case when the detergents available in the market are not sufficient and it is necessary to develop and synthesize new kinds of detergents [Lee et al. (2013), PNAS, www.pnas.org/cgi/doi/10.1073/pnas.1221442110]. In addition, all this requires a considerable amount of membrane proteins which in many cases (especially human proteins) are unavailable in large amounts. Other issue associated to the MP solubilization step with detergent is that detergents do not fully mimic biological membranes and in spite of decades of effort the process of finding the right detergent and the correct conditions for crystallization remains empirical and extremely laborious. A solution of this problem was not proposed within the LCP approach. In general, the LCP approach did not propose a new conception of the detergents in structural biology of membrane proteins.

Moreover, the original protocols defined by Landau and Rosenbusch (very low amount of the detergent and a low amount of protein buffer) became a dogma and have been applied in other different laboratories (*"As a rule of thumb, LCP and IAB may tolerate only 0.5-1.5% detergent by weight, depending upon the detergent that is being used"* see Rouhani, S. et al. Biopolymers 66, 300-316 (2002)), whereas these conditions appeared not to be appropriate to numerous membrane proteins; consequently, this approach led to a limited success of the LCP method.

Thus, a major drawback of the existing methods/approaches to membrane protein solubilization and crystallization is based on very laborious and expensive procedures of selection of a proper detergent for each particular membrane protein. This statement is equally applicable to LCP and in general to in meso approach.

The purpose of the present invention is to provide an easier and more reliable method that solves the problem linked to the selection of a detergent and makes rational the screening of crystallization conditions.

The Inventors have now developed a new method which solves the mentioned problems and allows one to use for crystallization any molecule belonging to a wide range of amphiphilic molecules: different detergents, surfactants, polymers, including those that usually denature proteins or could not be used for membrane proteins solubilization and crystallization. This method remarkably enhances efficiency of obtaining highest quality MP crystals, by applying a procedure of imbedding MPs (or MPs in membranes) into an initial crystallization matrix that forms interconnected amphiphilic bilayers (IAB) and the following crystallization by modulation of IAB properties (first of all the thickness of bilayer) via screening the concentration of the amphiphilic molecule; it significantly accelerates the process of obtaining proper crystals and is able to produce highest quality crystals for X-ray crystallography. The method is of high significance for structure based drug design since it provides crystals diffracting to high resolution.

The initial crystallization matrix is a bilayered membrane structure in which the membrane protein to be crystallized is introduced and embedded; it is composed of a host lipid capable to form a bicontinuous cubic and/or sponge phase; at least one buffer and optionally at least one amphiphilic molecule whose concentration influences at least the thickness of the IAB. Said initial crystallization matrix can be an interconnected bilayers structure but it is not necessary, it can also have a lamellar structure.

An essential feature of the present invention is that said initial crystallization matrix can form an interconnected amphiphilic bilayers system (lipidic sponge phase or lipidic cubic phase...) upon addition of a precipitant; the interconnection of the lipidic membranes of IAB is necessary to conduct the crystallization of membrane proteins according to the method of the present invention.

IAB is composed of at least a host lipid capable to form a bicontinuous cubic and/or sponge phase; at least one buffer; at least one amphiphilic molecule; at least one precipitant and at least one membrane protein.

The present invention thus relates to a new method of membrane protein crystallization that comprises the steps of:
(a) preparing at least one initial crystallization matrix capable to form interconnected amphiphilic bilayers (IAB) system by mixing, preferably via centrifugation or extrusion:
   - a host lipid capable to form a bicontinuous cubic and/or sponge phase;
   - optionally, at least one amphiphilic molecule whose concentration influences at least the thickness of the IAB;
   - at least one buffer;
      and adding at least one membrane protein; the membrane protein concentration is at least 5 mg/ml;
(b) initiating the crystallization of said at least one membrane protein by adding in the initial crystallization matrix at least one precipitant and at least one amphiphilic molecule if such amphiphilic molecule is not already present in the initial crystallization matrix prepared at step (a);
wherein the weight ratio host lipid/amphiphilic molecule is from 1:0,1 to 1:2,5 and the weight ratio host lipid/buffer is from 1:1 to 1:20.

Preferably said method comprises an additional step (c) that consists in repeating steps (a) and (b) at least 2 times with different concentrations of the at least one amphiphilic molecule.

The method is based on three discoveries.

The first one is that a key property of interconnected amphiphilic bilayer systems is the ability to embed fast MPs surrounded by different environments (solubilized in a detergent, or surrounded by an amphiphilic polymer, or embedded in biological or artificial lipid membranes, surfactants, bicelles, nanodiscs, etc).

The second is that a very important property of an integral bilayer which influences membrane protein behavior and may affect crystallization is the hydrophobic mismatch of the bilayer and the membrane protein; the mismatch induces stretching and bending of the bilayer around a protein and results in the excess of free energy due to elastic deformations of the bilayer tending to match the hydrophobic part of the protein. Inventors found out that optimal conditions of crystallization are determined by the thickness and bending rigidity of IAB more than by the type of the detergent; said thickness being modulated by concentration of the amphiphilic molecule.

The third one is that it is not necessary that initial crystallization matrix is the phase corresponding interconnected amphiphilic bilayers system (LCP, sponge, etc.); what really matters is that the initial crystallization matrix is capable to form interconnected amphiphilic bilayers upon addition of the precipitants.

The purpose of step (a) is to embed membrane proteins into the initial crystallization matrix that is capable to form an interconnected amphiphilic bilayer (IAB) matrix, membrane proteins being either in solubilized form or, preferably, in an amphiphilic environment where they are stable, such as directly from a membrane system: biomembranes (biological membranes or a patch of biological membranes containing a sufficient amount of the membrane protein of interest), lipid membranes, bicelles, nanodiscs. In such a bilayer membrane system, membrane protein can directly be embedded into initial crystallization matrix without prior solubilization. This step allows one to completely avoid solubilization of membrane proteins when said membrane protein of interest is, for example, poorly stable or present in a significant amount in the cell membranes and does not need usual steps of solubilization. Even when a solubilization is required, any mild proper detergent can be used for this purpose and the selection of such a detergent is determined by the solubilization criteria, and not by the crystallization conditions.

The person skilled in the art can easily determine temperature and pH to conduct steps (a) and (b) depending on the used host lipid; usually, temperature may be comprised between 5 and 25°C; for example, if monooleoyl is used as host lipid, the temperature will be comprised between 20 and 24°C.

In a particular embodiment of the present method, the membrane protein, in a membrane system or solubilized in a detergent, is added after the preparation of said initial crystallization matrix capable to form IAB consisting of the mixture of host lipid, optionally amphiphilic molecule, buffer. In such embodiment, step (a) comprise a step (a1) consisting in the preparation, preferably via centrifugation or extrusion, of a mixture comprising said host lipid, optionally said at least one amphiphilic molecule and said at least one buffer in the above ratios; and a step (a2) consisting in the addition of said membrane protein.

It is well known that membrane proteins may adopt several different conformations; some conformations result from the presence of a ligand (for example, native lipids of the considered membrane protein). In case a specific conformation is required to crystallize a specific membrane protein, one may add to the initial crystallization matrix the specific ligand that fixes said membrane protein in the desired conformation. As such ligand is specific to a given membrane protein, it is not possible to list them all but this can be found in the state of the art by a skilled person; for example, Cherezov et al. in (2007) Science 318, 1258 described the use of the ligand carazolol to crystallize the human beta2-adrenergic G protein-coupled receptor.

The host lipid can be a lipid which is capable to form a bicontinuous cubic phase and/or sponge phase; for example, monoglycerides including monooleoyl, monopalmytoyl, monovaccenin, isoprenoid-chained lipid β-XylOC₁₆₊₄.

The final concentration of the host lipid varies from 30 to 100%, preferably from 30 to 95%, more preferably from 30 to 90% and even more preferably from 50 to 95%, in weight relative to the total weight of host lipid and amphiphilic molecules.

An essential component of the method of the present invention is the amphiphilic molecule as it allows the modulation of the thickness and the rigidity of the lipid bilayer of the IAB; systematic studies of MPs crystallization from IAB conducted by Inventors (see crystallization experiments in the presence of the detergents belonging to different families and at different ratios of the polar and nonpolar fragments detailed in the examples) demonstrated a completely new role of detergent in this new crystallization method. Crystallization results together with the detailed X-ray and neutron diffraction characterization of the structure of IAB crystallization matrix demonstrated that crystallization of MPs from IAB is significantly driven by the thickness and rigidity of the lipid bilayer and the geometrical parameters of the mesophase, but not so much by the chemical structure of amphiphilic molecules (lipids, detergents...). The studies conducted by the Inventors show for the first time the possibility of crystallization of membrane protein in almost any types of detergents, both with short and long alkyl chains, and allowed to determine the quantitative characteristics, optimal for crystallization.

Moreover, the amphiphilic molecule according to the present invention may belong to a wide range of classes: detergents, surfactants, polymers, specific proteins (like a scaffold protein MSP1 used in nanodisc engeneering [T.H. Bayburt&S.G. Sligar(2010) FEBS Letters, 584, 1721-1727]) and peptides, like WALP or WT-20 from influenza X31 [Tenchov et al. (2013) Biophysical Journal 104, 1029-1037], including those that usually denature proteins or as it is believed cannot be used for solubilization and crystallization.

Additional examples of amphiphilic molecules are:
- n-alkyl-beta-D-glucopyranosides with different hydrocarbon chain lengths (from 6 to 12 carbon atoms);
- fluorinated surfactants (FS). Structurally FSs include of polar head group and a hydrophobic tail that consists of partially (like in case of hemifluorinated surfactants) or completely fluorinated chains; 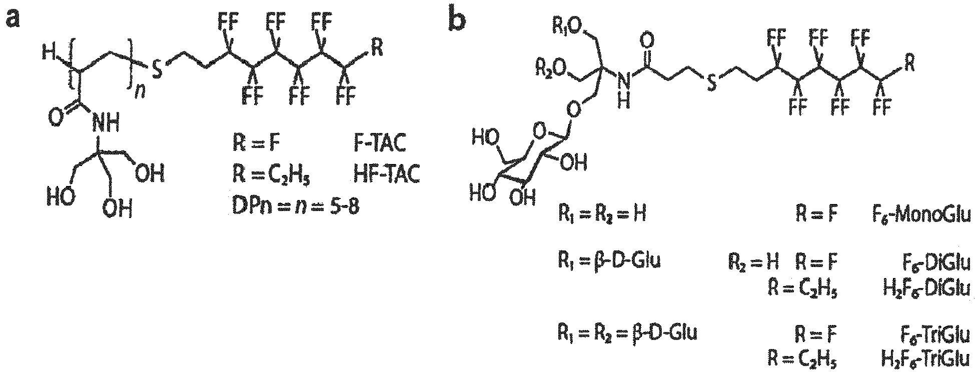 Chemical structure of some fluorinated surfactants. a) F-TAC and HF-TAC; b) (H)F-Mono-, Di- and TriGlu; F6- and H2F6-, refer to hydrophobic moieties, where R = F and R = C2H5, respectively [Popot,J.-L. (2010) Annu Rev Biochem 79, 737-775].
- Amphipoles [Y. Cohon&J.-L. Popot (2003) Curr. Opin. Colloid&Interface Science 8, 15-22];
- poloxamers: Poloxamers are a class of nonionic, surface-active amphiphilic polymers and can demonstrate more hydrophilic or hydrophobic properties depending on the ratio between their hydrophilic PEO blocks and hydrophobic PPO counterparts [Wang,J.Y. et al. (2012) Biomacromolecules 13(9), 2616-2623].

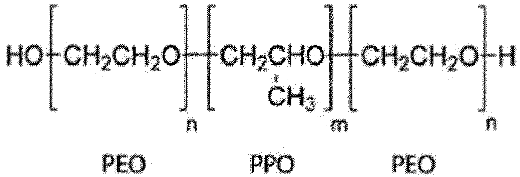

### Chemical structure of poloxamers

- surfactants (for instance, TOPO (trioctylphosphine oxide), which create inverse micelles (they have never been considered a suitable tool for membrane protein crystallization);
- a wide range of other detergents may be used, for example detergents sold under the commercial name of CYMAL including maltoside derivatives for example cyclohexyl-Methyl-β-D-maltoside; 2-cyclohexyl-1-ethyl-β-D-maltoside; 3-cyclohexyl-1-propyl-β-D-maltoside; 4-cyclohexyl-1-butyl-β-D-maltoside... (see also US Patent 5,674,987 and US Patent 5,763,586); or hydroxyethylglucamide derivatives, for example, cyclohexylbutanoyl-N-hydroxyethylglucamide (see Choudhury, D. et al. (1999) Science 285, 1061-1066) or alkyl glucoside derivatives such as 2,2-dioctylpropane-1,3-bis-β-D-maltopyranoside; 2,2-didecylpropane-1,3-bis-β-D-maltopyranoside; 2,2-dihexylpropane-1,3-bis-β-D-glucopyranoside; n-Octyl-β-D-galactoside... all these detergents are sold by Affymetrix (www.anatrace.com).

The amount of the amphiphilic molecules is comprised between 5 and 50% weight relative to the total weight of the initial crystallization matrix; this is much higher than the amount described in former *in meso* protocols/approaches that recommended a very low amount of the detergent, usually comprised between 0.5-1.5% detergent by weight since it was believed that large amounts of detergent deteriorate the LCP phase suitable for MPs crystallization.

The buffer conceptions are different from the existing ones. As in a standard case, the buffers must provide proper parameters (pH, type and concentration of the precipitants, etc.) consistent with protein stability and functionality. However, in addition, the buffers must satisfy new requirements; they should be able to dissolve the above mentioned amphiphilic molecules influencing the state of the crystallization phase, in particular, the thickness of the bilayer, which is used as a key screening parameter of the method of the invention.

The buffers that can be used in the method according to the present invention may be chosen amongst classical known buffers such as HEPES sodium salt [also named 4-(2-hydroxyethyl)piperazine-1-ethanesulfonic acid sodium salt or N-(2-hydroxyethyl)piperazine-N'-(2-ethanesulfonic acid) sodium salt]; imidazole; sodium acetate trihydrate (also named acetic acid sodium salt trihydrate); sodium cacodylate trihydrate (also named cacodylic acid sodium salt trihydrate or dimethylarsinic acid sodium salt or dimethylarsonic acid sodium salt); citrate tribasic dihydrate (also named citric acid trisodium salt dihydrate or trisodium citrate dehydrate); TRIS HCl (tris(hydroxymethyl)aminomethane hydrochloride)...

The recommended buffer content in the literature and in the known protocols is usually comprised from about 40 to 50% weight relative to the total weight of the initial crystallization matrix. This is a dramatic limitation. Inventors' studies of IAB crystallization diagrams showed that the optimal content of the buffer may be considerably larger. The mentioned above number can be as high as 90% or even higher. An optimal value depends on the membrane protein, the amphiphilic molecule, the amount of amphiphilic molecules and the host lipid and can be adjusted consequently. It should be mentioned that this value also depends on whether a dry or an aqueous precipitant is used. The amount of the buffer is an additional important screening parameter; it is comprised between 40 to 95% weight relative to the total weight of the initial crystallization matrix. The content of the buffer is specific for a given membrane protein and is classically defined by the experimentalist.

Optionally, buffers may also contain small amounts, less than 0,5 M, of salts (for example Na/K-Pi) and/or of PEGs.

Phase equilibrium of the crystallization system is usually reached within 1 to 24 hours; at equilibrium, membrane proteins are embedded in the bilayers of the initial crystallization matrix.

The step (b) consists of the crystallization of the membrane proteins embedded in IAB by the addition of at least one precipitant; usually precipitants are dry salts, for instance Na/K-Pi, or water solutions containing different salts like, for instance Na/K-Pi; said salts are added to the initial crystallization matrix in an amount comprised between about 0.5 and 4M; optionally, the precipitant comprises other additives such as, for instance, PEGs (200, 400, 600, 1000, 1500) or 2-methyl-2,4-pentadiol (MPD) from 10 to about 45% in weight compared to the total weight of precipitant.

The total volume of the precipitant if in the form of a water solution is usually varied from 50 to 95% from total weight of the initial crystallization matrix. If precipitant is used in dry form (i.e. dry salt), it is added to the initial crystallization matrix so that the final concentration of the salt in said initial crystallization matrix is comprised between 0,5 and 4 M.

As previously explained, the essential feature of the method according to the present invention is the IAB thickness that needs to be adjusted to the membrane protein of interest; in a preferred embodiment, the IAB thickness is modulated to obtain the optimized conditions in which the MP of interest crystallizes in a larger size and a better quality; the modulation of this parameter is obtained by screening the concentrations of said amphiphilic molecule added to IAB. This screening of amphiphilic molecule concentrations is performed by the repetition of the method of the invention several times.

Accordingly, in a preferred embodiment of the invention, the method is conducted several times, for example 96 times if the experiment is conducted in a 96-well plate, with different concentrations of the at least one amphiphilic molecule; in this embodiment, the concentrations of said at least one amphiphilic molecule are such as the weight ratio host lipid / amphiphilic molecule being from 1:0 and 1:2,5 being understood that at least one of said methods is such as the weight ratio host lipid / amphiphilic molecule is from 1:0,1 and 1:2,5.

Preferably, said method is conducted several times, for instance between 2 and 100, preferably between 24 and 96; more preferably, it is conducted several time, with two or more different host lipids and with different concentrations of the same amphiphilic molecule or the same mixture of amphiphilic molecules.

Once crystallized, membrane proteins crystals are observed and tested by classical techniques known by person skilled in the art, such as X-ray.

The present invention also relates to a kit for preparing an initial crystallization matrix comprising:
- a host lipid;
- optionally at least one amphiphilic molecule;
- at least one buffer;
- and instructions;
wherein instructions teach that the use of the weight ratio host lipid/amphiphilic molecule, if any, is from 1:0,1 to 1:2,5 and the weight ratio host lipid/buffer is from 1:1 to 1:20.

The present invention also relates to a kit of crystallization for conducting the method of the invention comprising:
- a host lipid;
- at least one amphiphilic molecule;
- at least one buffer;
- at least one precipitant;
- and instructions;
wherein instructions teach that the use of the weight ratio host lipid/amphiphilic molecule is from 1:0,1 to 1:2,5 and the weight ratio host lipid/buffer is from 1:1 to 1:20.

Nature of host lipid, amphiphilic molecule, buffers and precipitant are as previously described; said kit contains enough of each ingredients to be able to conduct the method of the invention.

Preferably, said kit for preparing an initial crystallization matrix and said kit of crystallization also comprise containers in which preparing initial crystallization matrix and IAB and in which conducting the step of crystallization.

The present invention further relates to a composition consisting in a preformed initial crystallization matrix composed of said host lipid, said at least one amphiphilic molecule and said at least one buffer in the above ratios and obtained by mixing those ingredients preferably via centrifugation or extrusion.

Another object of the present invention relates to a kit comprising several preformed initial crystallization matrix with different concentrations of amphiphilic molecule.

Another object of the invention relates to kits comprising preformed initial crystallization matrix and several concentrations of at least one precipitant to screen several conditions of crystallization.

The present invention also relates to an efficient kit of precipitants that comprises:
- at least one amphiphilic molecule;
- at least one buffer;
- at least one precipitant;
- and instructions;
wherein instructions teach that the use of the weight ratio amphiphilic molecule/buffer is from 1:20 to 1:1.

Said kit of precipitants allows the preparation of compositions of precipitant comprising at least one amphiphilic molecule; at least one buffer; and at least one precipitant. The composition of precipitant may be in water solution or in dry form.

Again, the nature of amphiphilic molecule, buffer and precipitant are as previously described and said kit and composition of precipitant contain enough of each ingredients to be able to conduct the method of the invention.

The physical-chemical parameters of the precipitant are, for instance, as described in "Sparse Matrix Sampling: a screening method for crystallization of proteins" by Jancarik, J. and Kim, S.H. J. Appl. Cryst., 24,409-411, 1991 ; the content of precipitant in the kit is so that the concentration of precipitant in the IAB prepared with said kit is comprised between 0,5 and 4 M.

A key and important specific feature of membrane proteins is that they have both polar and hydrophobic parts. In the case of water soluble proteins (WSPs) the surface of the proteins is polar. The existing kits for WSPs crystallization comprise different ingredients in different combinations to be able to favor protein crystallization by screening crystallization conditions (pH, type of ions, concentration of the salts, additives, etc.) through the modification of the polar surfaces of the proteins and water properties of the precipitant. An example of a WSPs kit is the Crystal Screen from Hampton Research containing three major components - salt, buffer, precipitant.

In accordance with all that is known from successful membrane protein crystallization, specific contacts between the proteins are usually also established through polar parts of MPs. Therefore, a necessary requirement for an efficient MPs crystallization kit is that it also satisfies similar to WSPs kits requirements.

However, such requirements are necessary, but not sufficient to create an optimal for MPs crystallization. Indeed, as previously mentioned, the interactions of MPs embedded into IAB are determined not only by direct interactions (for instance, electrostatic, hydrogen bonds) but also are determined by indirect interactions mediated by the lipid bilayer (hydrophobic mismatching of the bilayer and the protein) which depend to a large extent on the bilayer thickness.

Therefore, the second requirement is that the kits of precipitants must contain amphiphilic molecules in different concentrations to screen (modulate) the properties of the bilayer.

Another object of the invention relates to the use of kit of crystallization or of kit of precipitant to screen for optimal concentration of amphiphilic molecule allowing the determination of the optimal thickness of IAB.

Preferably, said kit of precipitants allows the preparation of several compositions of precipitant each containing different concentration of the amphiphilic molecule or mixture of amphiphilic molecules.

As a consequence, each of these kits may be proposed in a set that allows the preparation of different thicknesses of the IAB and, thus will allow performing efficient screening of crystallization conditions via gradual variation of membrane thickness.

A specific example of said kit of precipitants is a classical WSPs kit (containing three major screening components - salt, buffer, precipitant see Sparse Matrix Sampling: a screening method for crystallization of proteins. Jancarik, J. and Kim, S.H. J. Appl. Cryst., 24,409-411, 1991) to which is added an amphiphilic molecule, for instance, at least one of n-octyl-β-D-glucopyranoside and n-alkyl-β-D-glucopyranoside (n = 6, 7, 8, 9, 10, 12). Thus, major principle difference between existing kits and new one is the presence of an amphiphilic molecule which is able to regulate the thickness of the IAB matrix. The concentration of the amphiphilic molecule is an additional screening parameter of the crystallization conditions.

It is also important that this approach may profit from exploiting different host lipids which have different properties (first of all different lengths of hydrophobic tails). For those of these lipids which differ in the length very much the sets of the kits as described above are important to cover gradually a larger interval of the properties of the IAB crystallization matrix (first of all, the thickness of the bilayer).

The described above sets of kits of precipitants are very valuable for nano/pico-volume crystallization since there are no tools to preform very small amounts (nanovolumes) of crystallization matrix, each in different compositions.

As a consequence, another embodiment of the invention relates to a crystallization tool or device comprising:
- a crystallization plate with several recipients; such as a plate of 96 wells; in case said device is used for nano or pico volume crystallization, then the volume of each recipient is comprised between 500 nl to 100 µl so that each experiment is conducted with a volume of IAB comprised between 10 nl to 1 µl; but crystallization devices according to the present invention may also comprise larger volumes of recipient.
- each recipient containing at least one precipitant either in water solution or in dry form; the content of precipitant is so that it allows the preparation of IAB having a precipitant concentration comprised between 0,5 and 4 M.

Optionally, said recipients also comprise different concentrations of at least one amphiphilic molecule; said concentrations are chosen to be able to prepare initial crystallization matrixes comprising from 0 to 50%, preferably from 5 to 50%, of amphiphilic molecules in weight in reference to the total weight of initial crystallization matrix; and/or and a buffer.

Nature of amphiphilic molecule, buffer and precipitant are as previously described.

In a preferred embodiment, the crystallization device comprises:
- a crystallization plate with several recipients;
- each recipient containing:
   * at least one precipitant in dry form;
   * at least one amphiphilic molecule in a concentration chosen to be able to prepare initial crystallization matrixes comprising from 0 to 50%, preferably 5 to 50%, of said amphiphilic molecule in weight in reference to the total weight of initial crystallization matrix; and
   * at least one buffer.

The device of the invention is advantageous used to prepare IAB by introducing, in said recipient, initial crystallization matrix comprising membrane protein to be crystallized; as a consequence, the content of precipitant in each recipient is such that its final concentration in the IAB is comprised between 0,5 and 4 M.

An example of one recipient may be prepared as follows: aqueous precipitants (for instance, with the content described above) is loaded onto a crystallization plate. Then water evaporates. The amount of the precipitant loaded onto the plate is chosen taking into account following requirement: after loading of IAB matrix with the protein onto the top of dry precipitant the final concentration of the dissolved precipitant must be equal to that of the standard aqueous precipitant. For example, if the lipid matrix contains 50 nl of water then 50 nl of the precipitant must be used for preparation a set of dry precipitant.

Preferably, the crystallization device is used to conduct the method at nano or pico volumes; in such embodiment, the crystallization device is prepared from the described above precipitant in the form of water solutions containing different salts and buffers themselves that are introduced in an amount comprised between 30 and 800 nl in each recipient and that is then dried by evaporation of water. In addition, such a crystallization device for nano-pico volume or micro crystallization can be prepared by dispensing the mentioned above aqueous precipitants with the following evaporation of aqueous solution. The performed experiments showed that such a dry precipitant approach is complementary to the standard one and considerably increases the chances to obtain high quality membrane protein crystals.

### FIGURES

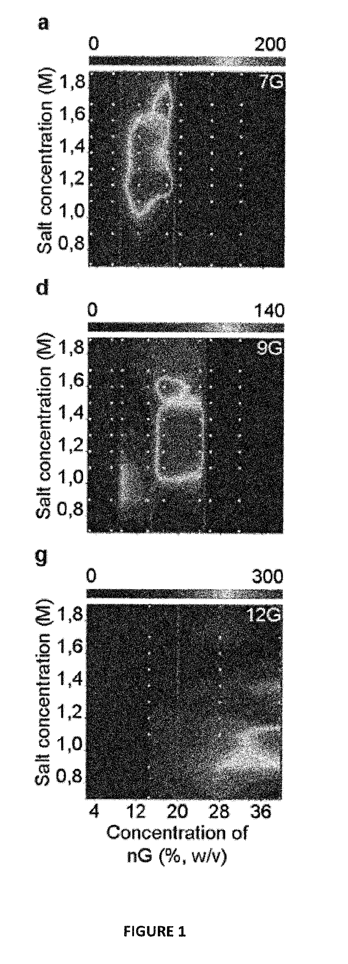

Figure 1 represents crystallization diagrams of BR in presence of different detergents (1:2 the MO/protein buffer ratio). The ordinate is concentration of dry powder Na/K-Pi salt in Mol, while the abscissa represents concentration (%) of a, n-heptyl-β-D-glucopyranoside (7G), d, n-nonyl-β-D-glucopyranoside (9G), g, n-dodecyl-β-D-glucopyranoside (12G). Contours are size of the BR crystals in micrometers.

### EXAMPLE: identification of high-resolution structures of new membrane proteins

The complex of two proteins sensory rhodopsin II and its cognate transducer and the mutants of this complex (NpSRII+NpHtrII) and other membrane proteins like a proton pump bacteriorhodopsin from *Halobium salinarum,* a chloride pump halorhodopsin, a proton pump *proteorhodopsin* ESR from *Exiguobacterium sibiricum,* bifunctional enzyme IPCT-DIPPS, nitrate/nitrite sensor histidine kinase NarQ were obtained by the new method. The structures of MPs where standard methods failed were obtained (NpSRII+NpHtrII), ESR, IPCT-DIPPS, NarQ. In addition, the method provides considerably higher quality crystals than those obtained by the known methods (bacteriorhodopsin and its mutants, NpSRII). The latter is also of high significance since comprehensive knowledge of molecular mechanisms of protein function can be obtained only when high resolution structures are available. The same is true of structure based computer drug design. Moreover, this method allows one to obtain large high quality membrane protein crystals from nanovolume crystallization plates which allow one to collect a complete crystallographic data set with a single crystal.

### 1. Crystallization of BR in an IAB matrix composed of MO/n-octyl-β-D-glucopyranoside/buffer

### I.A. Materials & methods

Preparation of Purple Membranes (PM) was done as it is described in details in Gordeliy et al. (2003) [Crystallization in lipidic cubic phases: A case study with Bacteriorhodopsin.In Membrane Protein Protocols: Expression, Purification, and Crystallization (Selinsky, B., ed), pp. 305-316, Humana Press, Totowa NJ].

The protocols are as follows.

### Cultivation of Halobacterium salinarum:

Pepton medium: 10 g/L pepton (1%; L37 from Oxoid), 250 g/L NaCl (4.3 *M*), 20 g/L MgSO4·7H2O (80 m*M*), 2 g/L KCl (27 m*M*), 3 g/L Na3citrate·2H2O (10 m*M*), adjust to pH 6.5 with NaOH. Sterilize for 2 h at 90°C in a dry sterilizer.

### Isolation of Purple Membrane

Basal salt solution: 250 g/L NaCl (4.3 *M*), 20 g/L MgSO4•7H2O (80 m*M*), 2 g/L KCl (27 m*M*).

### Transformation of Halobacterium salinarum

### 1. Support medium.

a. Dissolve 5 g tryptone and 3 g yeast extract in 200 mL H2O and autoclave to sterilize.
b. Dissolve 250 g NaCl, 20 g MgSO4·7H2O, 3 g Na3citrate·2H2O, 2 g KCl, 25 Ml 2 M Tris-HCl-pH 7.4 in 897 mL H2O and autoclave to sterilize.
c. 500 m*M* CaC12.
d. Mix components (a) and (b) and supplement with 3.1 mL component (c) to give a volume of 1.1 L.

### 2. Support medium agar plates.

a. Mix 5 g tryptone, 3 g yeast extract and 15 g agar with 200 mL H2O in a 1L bottle and dissolve slowly in a microwave oven.
b. Dissolve 250 g NaCl, 20 g MgSO4•7H2O, 3 g Na3citrate·2H2O, 2 g KCl, 25 mL 2 M Tris-HCl pH 7.4 in 897 mL H2O, and autoclave to sterilize.
c. 500 m*M* CaCl2.
d. Pour component (b) (cooled to 60°C after autoclaving) into the bottle with component (a) and supplement with 3.1 mL component (c). Depending on the shuttle vector add Novobiocin to a concentration of 1 g/mL or Mevinolin to a concentration of 50 M, mix well and pour into Petri dishes.

### 3. Spheroplast dilution solution:

Dissolve 250 g NaCl, 20 g MgSO4·7H2O, 3 g Na3citrate·2H2O, 2 g KCl, 25 mL 2 *M*Tris-HCl pH 7.4, 3.1 mL 500 m*M* CaCl2, and 150 g sucrose in 1 L H2O and filter sterilize.

### 4. Spheroplast buffer (SPH):

Dissolve 11.6 g NaCl (2 *M*), 0.2 g KCl (25 m*M*), 5 mL 1 *M*Tris-HCl pH 8.75 (50 m*M*) and 15 g sucrose in 100 mL H2O and filter sterilize.

### 5. Ethylanediaminetetraacetic acid (EDTA) solution: 500 mM EDTA in SPH, pH 8.75.

### 6. PEG solution: 6 mL PEG 600 in 4 mL SPH.

### 7. Regeneration medium: Supplement support medium with 15% sucrose and filter sterilize.

### Crystallization of Bacteriorhodopsin

Purple membranes (PM) were concentrated by centrifugation at 4000g. Then PM were transferred to crystallization buffer: 250 mMNa/K-Pi pH 5.6.

Monooleoyl (1-Monooleoyl-rac-glycerol, Sigma Chemicals) (MO) was melt at 40°C in 200 µL and spin down for 10 min at 13,000g at room temperature. Then PM solution in the buffer with 8% of n-octyl-β-D-glucopyranoside (14 mg/mL bR) was mixed 4 mg of MO. A homogenous lipid/protein mixture as well as the formation of the cubic phase aws achieved by the following centrifugation procedure. Spining of the PCR tube with the sample at 22°C at 10,000 rpm for 15 min. Rotate the tube within the rotor by 90° and spin again. Repeat this spinning procedure four times to obtain a homogenous mixture. The sample was kept for 24 h in the dark at 22°C. A grinded powder of KH2PO4 mixed with Na2HPO4 (95/5 w/w) to obtain a final concentration of 1-2.5 M phosphate (pH 5.6) was added to the PCR tube with the protein embedded into crystallization matrix. The sample was homogenized by repetitive centrifugation as described above. Crystallization batch was stored in the dark at 22°C. Crystals of bR usually appeared within 2-8 weeks after phosphate has been added

### I.B Results

The first experiments were done with n-octyl-β-D-glucopyranoside. These experiments showed that BR from PM is reconstituted into MO IAB ; under all tested MO/detergent (from 1:0.1 to 1:2.5) and MO/buffer (from 1:1 to 1:20) weight ratios. This is also supported by the fact that hexagonal plate-like crystals of the same P6₃ space group are grown under all the conditions. In addition, the samples are jelly-like, transparent and isotropically viscous. These are characteristic features of LCP and sponge (*L₃*) phases. X-ray diffraction directly supports this conclusion. The protein is stable under all conditions. It should be mentioned that the experiments with NpHtrII/transducer complex gave similar results.

These series of experiments showed that a considerably larger amount of the buffer can be used in IAB crystallization and it can be more favorable for growing high quality membrane protein crystals than in the published LCP protocols. Usually, the amount of protein solution is 40% in the final mixture with a matrix lipid [Caffrey, M. & Cherezov, V Nat. Protoc. 4, 706-731 (2009)]. This amount originated from the extremely important work on LCP crystallization of bacteriorhodopsin by Landau and Rosenbusch and was used by many researchers for similar studies [Landau, E.M. & Rosenbusch, J.P. Proc. Natl. Acad. Sci. USA. 93, 14532-14535 (1996)]. In the standard LCP crystallization method, described by Landau & Rosenbush, MO is mixed with the solubilized protein in aqueous solution; the resulting protein-containing cubic phase is then placed in contact with precipitant solution.

### II. Crystallization of BR in a IAB matrix composed of MO/n-alkyl-β-D-glucopyranoside/buffer

### II.A. Materials & methods

Materials and methods used in this assay are as in the case of crystallization of BR in an IAB matrix composed of MO/n-octyl-β-D-glucopyranoside/buffer described above. The only difference is that n-alkyl-β-D-glucopyranosides with different hydrocarbon chain lengths (n = 6, 7, 8, 9, 10, 12) were used instead of n-octyl-β-D-glucopyranoside.

### II.B Results

The second series of experiments was done with n-alkyl-β-D-glucopyranosides with different hydrocarbon chain lengths (n = 6, 7, 8, 9, 10, 12). BR successfully reconstitutes into the MO/detergent IAB in all the cases and for all the MO/buffer ratios (1:2, 1:2.5 and 1:3). It is important to note that the functionality of the protein is not reduced even with short hydrocarbon chains (n = 6 and 7).

Short hydrocarbon chain detergents are not suitable for standard solubilization, as BR loses the retinal. This means that 'solubilization' of BR from PM in the present assays is not proceeding through standard detergent micellar solubilization but likely by fision of PM with IAB of MO system (for instance, membranes of LCP) via a well known mechanism of lamellar to nonlamellar phase transition which involves characteristic topological intermediate states - 'inverted micellar intermediates' and 'interlamellar attachements' [Seddon, J.M. & Templer, R.H. Polymorphism of Lipid-Water Systems, from the Handbook of Biological Physics, Vol. 1, ed. R. Lipowsky, and E. Sackmann, Elsevier, Amsterdam, 97-160 (1995)].

In this new approach, the problem related to conflicting requirements for the properties of the detergents (imposed by the need for the two different processes: solubilization and crystallization) is completely solved - any 'soft' detergent can be used for solubilization. Moreover, the following reconstitution of the protein into a lipid membrane mimic system (lipid bilayers, bicelles, nanodiscs, polymers, like amphipoles which stabilize membrane proteins) will eliminate direct (very often disturbing) influence of this detergent on a protein and the following protein crystallization.

Practically any detergent/surfactant (if any is necessary) can be used for further crystallization from the membranes. After successful reconstitution of membrane protein into the MO/n-alkyl-β-D-glucopyranoside IAB BR crystals (hexagonal plates) grew with all detergents within 0.5 - 3 months. The growth time depends on detergent concentration: the larger the detergent concentration the longer the time required for growing crystals. For the MO/buffer ratios (1:2, 1:2.5 and 1:3) the observations are as follows. At larger ratios of the protein buffer to MO the maximum size of the crystals corresponds to higher concentration of the detergent (crystallization was done at a constant concentration of the protein).

The results can be represented as crystallization contour diagrams: size of the obtained BR crystals vs. concentration of the precipitant for all concentrations of each n-alkyl-β-D-glucopyranosides **(****Figure 1a, d, g****).** The optimum of protein crystallization (the darkest central part of the stains on the diagrams) corresponds to the larger crystals usually diffracting to higher resolution. From both sides of the optimum the size of the crystals gradually decreases and under certain deviations from the optimum the crystals do not grow at all. Fuzzy boundaries of the diagrams are explained by ∼10% experimental accuracy of the amount of the protein buffer, and the corresponding lipid/detergent ratio and a limited number of relatively large steps in the detergent concentrations screen. It was verified by checking reproducibility of the crystallization with n-octyl-β-D-glucopyranoside. When the diagrams for different lengths of the chains of the n-alkyl-β-D-glucopyranosides are compared then a rule can be derived: the optimum detergent concentration for crystal growth is shifting in a monotonic way with the length of the chain of the detergents: lower optimal concentrations correspond to shorter chains of the detergent, higher concentrations of the detergents correspond to longer chains. These results show that crystallization is driven by the thickness of the bilayers of IAB, not by the type of the detergent.

## Claims

1. Method of crystallization of membrane proteins that comprises the steps of:
(a) preparing at least one initial crystallization matrix capable to form interconnected amphiphilic bilayers (IAB) by mixing:
. a host lipid capable to form a bicontinuous cubic and/or sponge phases;
. optionally, at least one amphiphilic molecule;
. at least one buffer;
and adding at least one membrane protein;
(b) initiating the crystallization of said at least one membrane protein by adding in the initial crystallization matrix at least one precipitant and at least one amphiphilic molecule if such amphiphilic molecule is not already present in the initial crystallization matrix; wherein the weight ratio host lipid/amphiphilic molecule is from 1:0,1 to 1:2,5 and the weight ratio host lipid/buffer is from 1:1 to 1:20.

2. Method of crystallization of membrane proteins that comprises the steps of:
(a) preparing at least one initial crystallization matrix capable to form interconnected, amphiphilic bilayers (IAB) by mixing:
. a host lipid capable to form a bicontinuous cubic and/or sponge phases;
. optionally, at least one amphiphilic molecule;
. at least one buffer;
and adding at least one membrane protein;
(b) initiating the crystallization of said at least one membrane protein by adding in the initial crystallization matrix at least one precipitant and at least one amphiphilic molecule if such amphiphilic molecule is not already present in the initial crystallization matrix; wherein the weight ratio host lipid/amphiphilic molecule is from 1:0,1 to 1:2,5 and the weight ratio host lipid/buffer is from 1:1 to 1:20;
and (c) repeating steps (a) and (b) at least 2 times with different concentrations of the at least one amphiphilic molecule.

3. The method according to anyone of the preceding claims, wherein it is conducted several times with two different host lipids and different concentrations of the at least amphiphilic molecule.

4. The method according to anyone of the preceding claims, wherein said membrane protein is contained in a membrane system.

5. The method according to anyone of the preceding claims, wherein the host lipid is chosen amongst monoglycerides including monooleoyl, monopalmytoyl, monovaccenin, isoprenoid-chained lipid β-XylOC₁₆₊₄.

6. The method according to anyone of the preceding claims, wherein the amphiphilic molecule is chosen amongst detergents such as scaffold protein MSP1 and peptides like WALP or WT-20; n-alkyl-beta-D-glucopyranosides; surfactants such as standard or specific surfactants like fluorinated surfactants or those which create inverse micelles such as TOPO, amphiphilic polymers such as poloxamers; amphipoles; classical crystallization detergents such as maltoside derivatives; or hydroxyethylglucamide derivatives or alkyl glucosides derivatives.

7. The method according to any of the preceding claims, wherein a precipitant is a dry salt or a water solution containing at least one salt.

8. Kit for preparing an initial crystallization matrix comprising:
. a host lipid;
. optionally at least one amphiphilic molecule;
. at least one buffer;
. and instructions;
wherein instructions teach that the use of the weight ratio host lipid/amphiphilic molecule, if any, is from 1:0,1 to 1:2,5 and the weight ratio host lipid/buffer is from 1:1 to 1:20.

9. The kit for preparing an initial crystallization matrix according to claim 8, wherein it also comprises containers.

10. Composition consisting in a preformed initial crystallization matrix composed of:
. a host lipid;
. optionally at least one amphiphilic molecule;
. at least one buffer;
wherein the weight ratio host lipid/amphiphilic molecule, if any, is from 1:0,1 to 1:2,5 and the weight ratio host lipid/buffer is from 1:1 to 1:20.

11. Kit of crystallization comprising:
. a host lipid;
. at least one amphiphilic molecule;
. at least one buffer;
. at least one precipitant;
. and instructions;
wherein instructions teach that the use of the weight ratio host lipid/amphiphilic molecule is from 1:0,1 to 1:2,5 and the weight ratio host lipid/buffer is from 1:1 to 1:20.

12. The kit of crystallization according to claim 11, wherein it also comprises containers.

13. Kit of precipitants comprising:
. at least one amphiphilic molecule;
. at least one buffer;
. at least one precipitant;
. and instructions;
wherein instructions teach that the use of the weight ratio amphiphilic molecule/buffer is from 1:20 to 1:1.

14. Crystallization device comprising:
- a crystallization plate with several recipients;
- each recipient containing at least one precipitant in water solution or in dry form.

15. The crystallization device according to claim 14 comprising:
- a crystallization plate with several recipients;
- each recipient containing:
* at least one precipitant in dry form;
* at least one amphiphilic molecule in a concentrations chosen to be able to prepare initial crystallization matrixes comprising from 0 to 50%, preferably 5 to 50%, of amphiphilic molecules in weight in reference to the total weight of initial crystallization matrix; and
* at least one buffer.
